# EUROPÄISCHE PATENTSCHRIFT

(11) **EP 0 675 882 B1**
(45) Veröffentlichungstag und Bekanntmachung des Hinweises auf die Patenterteilung: **14.11.2001**
(21) Anmeldenummer: 94902743.7
(22) Anmeldetag: 10.12.1993
(51) Int. Cl.: C07D 279/06, C07D 417/04, C07D 417/12, A01N 43/86

(54) **5-ARYL-1,3-THIAZIN DERIVATE, DEREN HERSTELLUNG UND DEREN VERWENDUNG ALS SCHÄDLINGSBEKÄMPFUNGSMITTEL**
5-ARYL-1,3-THIAZINE DERIVATIVES, MANUFACTURE THEREOF AND USE AS PESTICIDES
DERIVES DE 5-ARYL-1,3-THIAZINE, LEUR PRODUCTION ET LEUR UTILISATION COMME PESTICIDES

(30) Priorität: 23.12.1992 DE 4243818
(43) Veröffentlichungstag der Anmeldung: 11.10.1995
(73) Patentinhaber: BAYER AG, 51368 Leverkusen (DE)
(72) Erfinder: FISCHER, Reiner, D-40789 Monheim (DE); LIEB, Folker, D-51375 Leverkusen (DE); RUTHER, Michael, D-40789 Monheim (DE); STETTER, Jörg, D-42115 Wuppertal (DE); ERDELEN, Christoph, D-42799 Leichlingen (DE); WACHENDORFF-NEUMANN, Ulrike, D-40789 Monheim (DE); DOLLINGER, Markus, D-42799 Leichlingen (DE); LÜRSSEN, Klaus, D-51469 Bergisch Gladbach (DE); SANTEL, Hans-Joachim, D-51371 Leverkusen (DE)
(86) Internationale Anmeldenummer: EP9303483
(87) Internationale Veröffentlichungsnummer: WO9414785

(56) Entgegenhaltungen:
- EP-A- 0 010 420
- EP-A- 0 245 901
- EP-A- 0 503 958
- JOURNAL OF HETEROCYCLIC CHEMISTRY Bd. 10, Nr. 2 , April 1973 Seiten 223 - 224 KETCHAM R. ET AL. 'Synthesis of heterocycles. 174. Substituted thiazines and bisthiazinyls from dithiooxamide and trichlorophenyl malonates' in der Anmeldung erwähnt
- MONATSHEFTE FüR CHEMIE Bd. 95, Nr. 1 , 1964 Seiten 147 - 155 ZIEGLER E. & STEINER E. 'Synthesen von Heterocyclen, 52. Mitt.: Über Derivate des 2-Phenyl-4-hydroxy-[1,3-thiazinons-(6)]' in der Anmeldung erwähnt

## Beschreibung

Die Erfindung betrifft neue 5-Aryl-1,3-thiazin-Derivate, Verfahren zu ihrer Herstellung und ihre Verwendung als Schädlingsbekämpfungsmittel.

Bestimmte im Phenylring unsubstituierte 5-Phenyl-1,3-thiazin-Derivate sind bereits bekannt geworden (vgl. E. Ziegler und E. Steiner, Monatsh. 95, 147 (1964), R. Ketcham, T. Kappe und E. Ziegler, J. Heterocycl. Chem. 10, 223 (1973)), wobei für diese Verbindungen eine mögliche Anwendung als Schädlingsbekämpfungsmittel nicht angegeben wird.

Es wurden nun die neuen substituierten 5-Aryl-1,3-thiazin-Derivate der allgemeinen Formel (I) 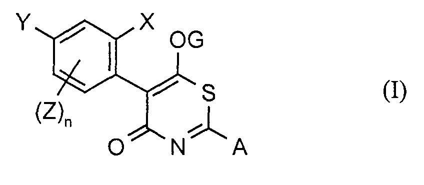 gefunden, in welcher
- A: für gegebenenfalls durch Halogen substituierte Reste aus der Reihe C₁-C₈-Alkyl, C₃-C₈-Alkenyl, C₃-C₈-Alkinyl und C₃-C₈-Cycloalkyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyl, Naphthyl, Hetaryl, Phenyl-C₁-C₄-alkyl und Hetaryl-C₁-C₄-alkyl oder für gegebenenfalls durch Halogen substituierte Reste aus der Reihe C₁-C₆-Alkoxy, C₃-C₆-Alkenyloxy, C₃-C₆-Alkinyloxy und C₃-C₆-Cycloalkoxy; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyloxy, Naphthyloxy, Phenyl-C₁-C₂-alkoxy, Thienyloxy, Furyloxy, Thiazolyloxy, Pyridyloxy und Pyrazolyloxy oder für gegebenenfalls durch Halogen substituierte Reste aus der Reihe C₁-C₆-Alkylthio, C₃-C₆-Alkenylthio, C₃-C₆-Alkinylthio und C₃-C₆-Cycloalkylthio, für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenylthio, Naphthylthio, Phenyl-C₁-C₂-alkylthio, Thienylthio, Thienyl-C₁-C₂-alkylthio, Furylthio, Thiazolylthio, Pyridylthio und Pyrazolylthio oder für die Gruppen
COR¹, CSR¹, CO₂-R¹, 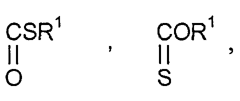 CS₂R¹, CN, CONR¹R² und CSNR¹R² steht, wobei
- R¹ und R²: unabhängig voneinander für gegebenenfalls durch Halogen substituierte Reste aus der Reihe C₁-C₆-Alkyl und C₃-C₆-Alkenyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituiertes Phenyl stehen,
- X: für Fluor, Chlor, Brom, Methyl, Ethyl, n- und i-Propyl, Methoxy oder Ethoxy steht,
- Y: für Wasserstoff, Fluor, Chlor, Brom, C₁-C₄-Alkyl oder Trifluormethyl steht,
- Z: für Wasserstoff, Fluor, Chlor, Brom, C₁-C₄-Alkyl, Methoxy oder Ethoxy steht,
- n: für 1 steht,
- G: für Wasserstoff, -COR³ oder COOR⁴steht,
- R³: für gegebenenfalls durch Fluor und/oder Chlor substituierte Reste aus der Reihe C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₁-C₄-alkyl und gegebenenfalls durch 1 oder 2 Sauerstoff- und/oder Schwefelatome unterbrochenes C₃-C₆-Cycloalkyl; für gegebenenfalls durch Fluor, Chlor, Brom, Nitro, C₁-C₃-Alkyl, Methoxy, Ethoxy, Trifluormethyl und/oder Trifluormethoxy substituiertes Phenyl, für gegebenenfalls durch Fluor, Chlor, Brom, C₁-C₃-Alkyl, Methoxy, Ethoxy, Trifluormethyl und/oder Trifluormethoxy substituiertes Phenyl-C₁-C₃-alkyl; für gegebenenfalls durch Fluor, Chlor, Brom, Methyl und/oder Ethyl substituierte Reste aus der Reihe Pyridyl, Pyrimidyl, Thiazolyl und Pyrazolyl;
- R⁴: für gegebenenfalls durch Fluor und/oder Chlor substituierte Reste aus der Reihe C₁-C₁₄-Alkyl, C₂-C₁₄-Alkenyl, C₁-C₄-Alkoxy-C₁-C₆-alkyl, C₁-C₄-Polyalkoxy-C₁-C₆-alkyl oder für gegebenenfalls durch Fluor, Chlor, Nitro, C₁-C₃-Alkyl, Methoxy, Ethoxy und/oder Trifluormethyl substituierte Reste aus der Reihe Phenyl und Benzyl steht,
wobei als Hetarylreste Pyrryl, Furyl, Thienyl, Thiazolyl, Pyridyl, Pyrazolyl und Pyrimidinyl in Frage kommen.

Die Verbindungen der allgemeinen Formel (I) können in Abhängigkeit der Substituenten als geometrische und/oder optische Isomere oder Isomerengemische unterschiedlicher Zusammensetzung vorliegen. Sowohl die reinen Isomeren als auch die Isomerengemische, deren Herstellung und Verwendung sowie diese enthaltende Mittel sind Bestandteile der beanspruchten Erfindung. Im folgenden wird der Einfachheit halber jedoch stets von Verbindungen der Formel (I) gesprochen, obwohl sowohl die reinen Verbindungen als gegebenenfalls auch Gemische mit unterschiedlichen Anteilen an isomeren Verbindungen gemeint sind.

Weiterhin wurde gefunden, dass die neuen Verbindungen der allgemeinen Formel (I) eine sehr gute Wirksamkeit als Schädlingsbekämpfungsmittel, vorzugsweise als Insektizide, Akarizide, Nematizide und Herbizide sowie als Ekto- und Endoparasitizide aufweisen.

Weiterhin wurde gefunden, dass man die neuen 5-Aryl-1,3-thiazin-Derivate der allgemeinen Formel (I) erhält, wenn man
a) zur Herstellung der Verbindungen, in welchen G für Wasserstoff steht,
   Thioamide der allgemeinen Formel (II) 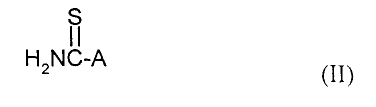 in welcher
   - A: die oben angegebene Bedeutung hat,
   mit Ketensäurehalogeniden der allgemeinen Formel (III) 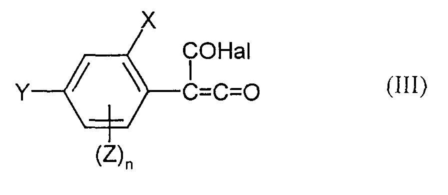 in welcher
   - X, Y, Z und n: die oben angegebenen Bedeutungen haben und
   - Hal: für Halogen (vorzugsweise für Chlor oder Brom) steht
   in Gegenwart eines Verdünnungsmittels und gegebenenfalls in Gegenwart eines Säureakzeptors umsetzt; und
b) zur Herstellung der Verbindungen der allgemeinen Formel (I), in welcher G für -COR³ steht,
   Verbindungen der allgemeinen Formel (I), in welchen G für Wasserstoff steht (erhältlich nach Variante a))
   α) mit Säurehalogeniden der allgemeinen Formel (IV) 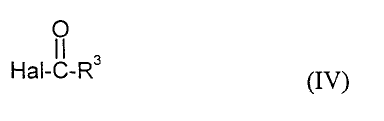 in welcher
      - R³: die oben angegebene Bedeutung hat und
      - Hal: für Halogen (vorzugsweise für Chlor oder Brom) steht, oder
   β) mit Carbonsäureanhydriden der allgemeinen Formel (V) 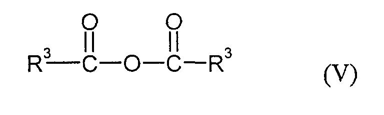 in welcher
      - R³: die oben angegebene Bedeutung hat,
      gegebenenfalls in Gegenwart eines Verdünnungsmittels und gegebenenfalls in Gegenwart eines Säurebindemittels umsetzt; und
   c) zur Herstellung der Verbindungen der allgemeinen Formel (I), in welchen G für COOR⁴ steht und R⁴ die oben angegebene Bedeutung besitzt,
      Verbindungen der allgemeinen Formel (I), in welchen G für Wasserstoff steht (erhältlich nach Variante a)),
      mit Verbindungen der allgemeinen Formel (VI) 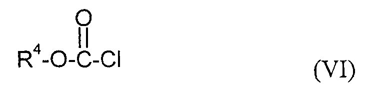 in welcher
      - R⁴: die oben angegebenen Bedeutungen hat,
      gegebenenfalls in Gegenwart eines Verdünnungsmittels und gegebenenfalls in Gegenwart eines Säurebindemittels umsetzt.

Bevorzugt sind Verbindungen der Formel (I) gemäß Anspruch 1, in welcher
- A: für gegebenenfalls durch Halogen substituierte Reste aus der Reihe Phenyl, Phenyloxy, Phenylthio steht,
- X: für Methyl steht,
- Y: für Methyl steht,
- Z: für Methyl steht,
- n: für 1 steht,
- G: für Wasserstoff steht.

Die oben aufgeführten allgemeinen oder im Vorzugsbereich aufgeführten Restedefinitionen bzw. Erläuterungen können untereinander beliebig kombiniert werden. Sie gelten für die Endprodukte sowie für die Vor- und Zwischenprodukte entsprechend.

Erfindungsgemäß bevorzugt werden die Verbindungen der allgemeinen Formel (I), in welchen eine Kombination der vorstehend als bevorzugt aufgeführten Bedeutungen vorliegt.

Die zur Durchführung der erfindungsgemäßen Verfahrensvariante b) und c) als Ausgangsstoffe benötigten 3-Aryl-1,3-thiazine sind nach Verfahrensvariante a) erhältlich.

Die zur Durchführung der erfindungsgemäßen Verfahrensvarianten b) und c) außerdem als Ausgangsstoffe benötigten Säurehalogenide der Formel (IV), Carbonsäureanhydride der Formel (V), Chlorameisensäureester oder Chlorameisensäurethioester der Formel (VI) sind allgemein bekannte Verbindungen der organischen bzw. anorganischen Chemie.

Auch die Thioamidverbindungen der Formel (II) sind allgemein bekannte Verbindungen in der organischen Chemie.

Die Verbindungen der Formel (III) sind teilweise bekannt (vgl. beispielsweise Org. Prep. Proced. Int., 7(4), 155-8, 1975 und DE 19 45 703). Die noch nicht bekannten Verbindungen lassen sich aber nach im Prinzip bekannten Methoden in einfacher Weise analog herstellen. So erhält man z.B. Halogencarbonylketene der Formel (III), wenn man Arylmalonsäuren der Formel (XIII) 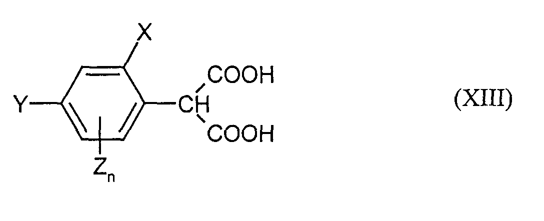 in welcher
X, Y, Z und n die oben angegebene Bedeutung haben,
mit Säurehalogeniden, wie beispielsweise Thionylchlorid, Phosphor(V)chlorid, Phosphor(III)chlorid, Oxalylchlorid, Phosgen oder Thionylbromid gegebenenfalls in Gegenwart von Katalysatoren, wie beispielsweise Diethylformamid, Methyl-Stearylformamid oder Triphenylphosphin, umsetzt.

Die Arylmalonsäuren der Formel (XIII) sind allgemein bekannte Verbindungen der organischen Chemie (vgl. z.B. Organikum, VEB Deutscher Verlag der Wissenschaften, Berlin 1977, S. 517 ff.).

Als Verdünnungsmittel können bei der erfindungsgemäßen Verfahrensvariante a) alle inerten organischen Solventien eingesetzt werden. Vorzugsweise verwendbar sind Kohlenwasserstoffe, wie Toluol und Xylol, ferner Ether, wie Dibutylether, Glykoldimethylether und Diglykoldimethylether, außerdem polare Lösungsmittel, wie Dimethylsulfoxid, Sulfolan, Dimethylformamid und N-Methyl-pyrrolidon.

Als Säureakzeptoren können bei der Durchführung der erfindungsgemäßen Verfahrensvariante a) alle üblichen Säureakzeptoren verwendet werden.

Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin.

Die Reaktionstemperaturen können bei der Durchführung der erfindungsgemäßen Verfahrensvariante a) innerhalb eines größeren Bereiches variiert werden. Zweckmäßigerweise arbeitet man bei Temperaturen zwischen 0°C und 250°C, vorzugsweise zwischen 20°C und 220°C.

Die erfindungsgemäße Verfahrensvariante a) wird zweckmäßigerweise unter Normaldruck durchgeführt.

Bei der Durchführung der erfindungsgemäßen Verfahrensvariante a) setzt man die Reaktionskomponenten der Formeln (II) und (III) und gegebenenfalls die Säureakzeptoren zweckmäßigerweise in etwa äquimolaren Mengen ein. Es ist jedoch auch möglich, die eine oder andere Komponente in einem größeren Überschuß (bis zu 5 Mol) zu verwenden.

Als Verdünnungsmittel können bei der erfindungsgemäßen Verfahrensvariante b) α) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach der erfindungsgemäßen Verfahrensvariante b) α) alle üblichen Säureakzeptoren in Betracht. Vorzugsweise verwendbar sind tertiäre Amine, wie Triethylamin, Pyridin, Diazabicyclooctan (DABCO), Diazabicycloundecan (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat sowie Alkalihydroxide, wie Natrium- und Kaliumhydroxid.

Die Reaktionstemperaturen können auch bei der erfindungsgemäßen Verfahrensvariante b) α) innerhalb eines größeren Bereiches variiert werden. Zweckmäßigerweise arbeitet man bei Temperaturen zwischen -20°C und +150°C, vorzugsweise zwischen 0°C und 100°C.

Bei der Durchführung der erfindungsgemäßen Verfahrensvariante b) α) werden die Ausgangsstoffe der Formeln (I) und das Carbonsäurehalogenid der Formel (V) vorzugsweise in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Verwendet man bei der erfindungsgemäßen Verfahrensvariante b) β) als Reaktionskomponente der Formel (V) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren. Auch als Säureakzeptoren können die bei der Verfahrensvariante b) α) angegebenen Säureakzeptoren eingesetzt werden.

Die Reaktionstemperaturen können bei der erfindungsgemäßen Verfahrensvariante b) β) auch bei der Verwendung von Carbonsäureanhydriden innerhalb eines größeren Bereiches variiert werden. Zweckmäßigerweise arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (I) und das Carbonsäureanhydrid der Formel (V) vorzugsweise in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Zweckmäßigerweise geht man so vor, daß man Verdünnungsmitel und im Überschuß vorhandenes Carbonsäureanhydrid sowie die entstehende Carbonsäure durch Destillation oder durch Waschen mit einem organischen Lösungsmittel oder mit Wasser entfernt.

Verwendet man bei der Verfahrensvariante c) die entsprechenden Chlorameisensäureester bzw. Chlorameisensäurethiolester so kommen als Säurebindemittel bei der Umsetzung alle üblichen Säureakzeptoren in Betracht.

Vorzugsweise verwendbar sind tertiäre Amine, wie Triethylamin, Pyridin, 1,4-Diazabicyclo[2.2.2]octan (DABCO), 1,8-Diazabicyclo[5.4.0]undec-7-en (DBU), 1,5-Diazabicyclo[4,3,0]non-5-en (DBN), Hünig-Base und N,N-Dimethyl-anilin, ferner Erdalkalimetalloxide, wie Magnesium- und Calcium-oxid, außerdem Alkali- und Erdalkalimetall-carbonate, wie Natriumcarbonat, Kaliumcarbonat und Calciumcarbonat sowie Alkalihydroxide, wie Natrium- oder Kaliumhydroxid.

Als Verdünnungsmittel können bei der erfindungsgemäßen Verfahrensvariante c) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Bei Verwendung der Chlorameisensäureester bzw, Chlorameisensäurethiolester als Carbonsäure-Derivate der Formel (VI) können die Reaktionstemperaturen bei der Durchführung der erfindungsgemäßen Verfahrensvariante c) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Die erfindungsgemäße Verfahrensvariante c) wird zweckmäßigerweise unter Normaldruck durchgeführt.

Bei der Durchführung der erfindungsgemäßen Verfahrensvariante c) werden die Ausgangsstoffe der Formel (I) und der entsprechende Chlorameisensäureester bzw. Chlorameisensäurethiolester der Formel (VI) vorzugsweise in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Zweckmäßigerweise geht man so vor, daß man ausgefallene Salze entfernt und das verbleibende Reaktionsgemisch durch Abziehen des Verdünnungsmittels einengt.

Die erfindungsgemäßen Verbindungen der Formel I können zur Schädlingsbekämpfung eingesetzt werden, Schädlinge sind unerwünschte tierische Schädlinge, insbesondere Insekten, Milben und Nematoden, welche Pflanzen oder höhere Tiere schädigen. Zu den Schädlingen gehören jedoch auch unerwünschte Pflanzen.

Die erfindungsgemäßen Wirkstoffe eignen sich bei guter Pflanzenverträglichkeit und günstiger Wärmeblütertoxizität zur Bekämpfung von tierischen Schädlingen, vorzugsweise von Anthropoden, insbesondere von Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.

Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psyllio des chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp,
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp,.
Aus der Ordnung der Arachnida z.B. Scorpio maurus, Latrodectus mactans,

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Daneben besitzen die erfindungsgemäßen Wirkstoffe der Formel (I) auch eine gute fungizide Wirksamkeit und lassen sich zur Bekämpfung von Pflanzenkrankheiten wie beispielsweise gegen den Erreger der Reisfleckenkrankheit (Pyricularia oryzae) einsetzen.

Die erfindungsgemäßen Wirkstoffe können zur Verwendung als Insektizide, Akarizide und Nematizide in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Verbindungen eignen sich auch in besonderer Weise zur Behandlung von vegativem und generativem Vermehrungsmaterial, wie z.B. von Saatgut von Getreide, Mais, Gemüse u.s.w. oder von Zwiebeln, Stecklingen u.s.w.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe können auch als Herbizide, vorzugsweise als Defoliants, Desiccants, Krautabtötungsmittel und insbesondere als Unkrautvernichtungsmittel verwendet werden, Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen Unkräutern in dikotylen Kulturen sowohl im Vorauflauf- als auch im Nachauflauf-Verfahren.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streu en.

Die erfindungsgemäßen Wirkstoffe können sowohl vor, als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Zur Herstellung der Schädlingsbekämpfungsmittel können die erfindungsgemäßen Wirkstoffe in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u,ä., sowie ULV-Kalt- und Warmneben-Formulierungen.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä, sowie ULV-Kalt- und WarmnebelFormulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.
Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff
und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage:
z.B, gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage:
z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Mittel enthalten bevorzugt neben wenigstens einer verbindung der allgmeeinen Formel (I) und gegebenenfalls neben erheblichen Streck- und Hilfsmitteln wenigstens einen oberflächenaktiven Stoff.

Die Wirkstoffe eignen sich bei günstiger Warmblütertoxizität auch zur Bekämpfung von tierischen Schädlingen (Ekto- und Endoparasiten) wie Arthropoden, vorzugsweise Insekten und Spinnentieren (Ektoparasiten), Cestoden, Trematoden, Nematoden und Acantocephalen (Endoparasiten), die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Durch die Bekämpfung der tierischen Schädlinge sollen Krankheiten und deren Übertragung, Todesfälle und Leistungsminderungen (z.B. bei der Produktion von Fleisch, Milch, Wolle, Häuten, Eiern) verhindert werden, so daß durch den Einsatz der Wirkstoffe eine wirtschaftlichere und einfachere Tierhaltung möglich ist bzw. in bestimmten Gebieten erst möglich wird.

Zu den Schädlingen gehören:
Aus der Ordnung der Anoplura z.B. Haematopinus spp., Linognathus spp., Solenopotes spp., Pediculus spp., Pthirus spp.;
aus der Ordnung der Mallophaga z.B. Trimenopon spp., Menopon spp., Eomenacanthus spp., Menacanthus spp., Trichodectes spp., Felicola spp., Damalinea spp., Bovicola spp;
aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oesteromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..

Aus der Ordnung der Metastigmata z.B. Hyalomma spp., Rhipicephalus spp., Boophilus spp., Amblyomma spp., Haemophysalis spp., Dermacentor spp., Ixodes spp., Argas spp., Ornithodorus spp., Otobius spp.;
aus der Ordnung der Mesastigmata z.B. Dermanyssus spp., Ornithonyssus spp., Pneumonyssus spp..

Aus der Ordnung der Prostigmata z.B. Cheyletiella spp., Psorergates spp., Myobia spp., Demodex spp., Neotrombicula spp,;
aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neoknemidocoptes spp. Lytodites spp., Laminosioptes spp..

Zu den Endoparasiten gehören:

Aus der Ordnung der Pseudophyllidea z.B.; Diphyllobothrium spp., Spirometra spp., Schistocephalus spp., Ligula spp., Bothridium spp., Diphlogonoporus spp..

Aus der Ordnung der Cyclophyllidea z.B.: Mesocestoides spp., Anoplocephala spp., Paranoplocephala spp., Moniezia spp., Thysanosomsa spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp..

Aus der Unterklasse der Monogenea z.B.: Gyrodactylus spp., Dactylogyrus spp., Polystoma spp..

Aus der Unterklasse der Digenea z.B.: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fasciolides spp., Fasciolopsis spp., Cyclocoelum spp., Typhlocoelum spp., Paramphistomum spp., Calicophoron spp-, Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp..

Aus der Ordnung der Enoplida z.B.: Trichuris spp., Capillaria spp., Trichomosoides spp., Trichinella spp..

Aus der Ordnung der Rhabditia z.B.: Micronema spp., Strongyloides spp..

Aus der Ordnung der Strongylida z.B.: Stronylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Bunostomum spp., Globocephalus spp., Syngamus spp., Cyathostoma spp., Metastrongylus spp., Dictyocaulus spp., Muellerius spp., protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Angiostrongylus spp., Aelurostrongylus spp., Filaroides spp., Parafilaroides spp., Trichostrongylus spp., Haemonchus spp., Ostertagia spp., Marshallagia spp., Cooperia spp., Nematodirus spp., Hyostrongylus spp., Obeliscoides spp., Amidostomum spp., Ollulanus spp..

Aus der Ordnung der Oxyurida z.B.: Oxyuris spp., Enterobius spp., Passalurus spp., Syphacia spp., Aspiculuris spp., Heterakis spp..

Aus der Ordnung der Spirurida z.B.: Gnathostoma spp., Physaloptera spp., Thelazia spp., Gongylonema spp., Habronema spp., Parabronema spp., Draschia spp., Dracunculus spp..

Aus der Ordnung der Filariida z.B.: Stephanofilaria spp., Parafilaria spp., Setaria spp., Loa spp., Dirofilaria spp., Litomosoides spp., Brugia spp., Wuchereria spp., Onchocerca spp..

Aus der Ordnung der Gigantorhynchida z.B.: Filicollis spp., Moniliformis spp., Macracanthorhynchus spp., Prosthenorchis spp..

Zu den Nutz- und Zuchttieren gehören Säugetiere wie z.B. Rinder, Pferde, Schafe, Schweine, Ziegel, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Süß- und Salzwasserfische wie z.B. Forellen, Karpfen, Aale, Reptilien, Insekten wie z.B. Honigbiene und Seidenraupe.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Zäpfchen, Tabletten, Kapseln, Pasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:
Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylakohol, Glycerin, Kohlenwasserstoffe, Propylenglykol, Polyethylenglykole, N-Methyl-pyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht. Diese Lösungen werden wie oben bei den Injektionslösungen beschrieben hergestellt.

Es kann vorteilhaft sein, bei der Herstellung Verdikkungsmittel zuzufügen. Verdickungsmittel sind: Anorganische Verdickungsmittel wie Bentonite, kolloidale Kieselsäure, Aluminiummonostearat, organische Verdikkungsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff entweder die Haut durchdringt und systemisch wirkt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschufzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methyl-pyrrolidon, 2,2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B, DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/ Caprinsäure-bigylcerid, Triglyceridgemisch mit Pflanzenfettsäuren der Kettenlänge C₈₋₁₂ oder anderen speziell ausgewählten natürlichen Fettsäuren, Partialglyceridgemische gesättigter oder ungesättigter eventuell auch hydroxylgruppenhaltiger Fettsäuren, Mono- und Diglyceride der C₈/C₁₀-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge C₁₆-C₁₈, Isopropylmyristat, Isopropylpalmitat, Capryl/Caprinsäureester von gesättigten Fettalkoholen der Kettenlänge C₁₂-C₁₈, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt:
nichtionogene Tenside, z.B, polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-ß-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die vorliegende Erfindung betrifft somit auch die Verbindungen der allgemeinen Formel (I) zur Verwendung als Ekto- und Endoparasitizide sowie die Verwendung der Verbindungen der allgemeinen Formel (I) zur Herstellung eines Mittels zur Bekämpfung von Ekto- und Endoparasiten.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenylimidazothiazol, Benzimidazolcarbamate, Praziquantel, Pyrantel, Febantel.

Anwendungsfertige Zubereitungen enthalten den Wirkstoff in Konzentrationen von 10 ppm - 20 Gewichtsprozent, bevorzugt von 0,1 - 10 Gewichtsprozent.

Zubereitungen die vor Anwendung verdünnt werden, enthalten den Wirkstoff in Konzentrationen von 0,5 - 90 Gewichtsprozent, bevorzugt von 5 bis 50 Gewichtsprozent.

Im allgemeinen hat es sich als vorteilhaft erwiesen, Mengen von etwa 1 bis etwa 100 mg Wirkstoff je kg Körpergewicht pro Tag zur Erzielung wirksamer Ergebnisse zu verabreichen.

Die erfindungsgemäßen Verbindungen werden bevorzugt als Arthropodizie und Herbizide in den Bereichen Pflanzenschutz, Haushalt- und Hygiene sowie im Vorratsschutz, ganz besonders bevorzugt im Pflanzenschutz eingesetzt.

Wo nicht anderes angegeben wird sind alle Prozentangaben Gewichtsprozente.

Die Herstellung der erfindungsgemäßen Verbindungen der allgemeinen Formel (I) soll durch die folgenden Herstellungsbeispiele und die biologische Wirksamkeit durch die folgenden biologischen Beispiele erläutert werden.

### Beispiel 1

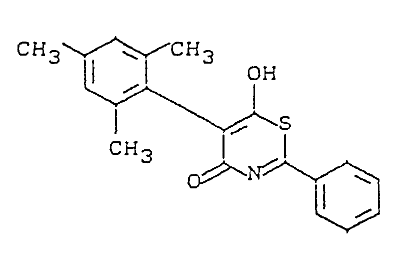

Zu 8,9 g (40 mmol) Chlorcarbonyl-2,4,6-trimethylphenylketen in 80 ml absolutem Toluol gibt man unter Feuchtigkeitsausschluß 5,5 g (40 mmol) Thiobenzamid bei 20°C und erwärmt 6 Stunden auf 50°C. Man trennt den Niederschlag ab, kristallisiert aus Ethylacetat um und erhält 5,1 g 6-Hydroxy-2-phenyl-5-(2,4,6-trimethylphenyl)-1,3-thiazin-4-on (Ausbeute: 39 % der Theorie). Fp. = 240 bis 242°C. ¹H-NMR (CD₃OD, TMS interner Standard): δ = 2,10 (s,6H), 2,26 (s,3H), 6,88 (s,2H, 7,56 (m,3H), 8,13 (m,2H).

### Beispiel 2

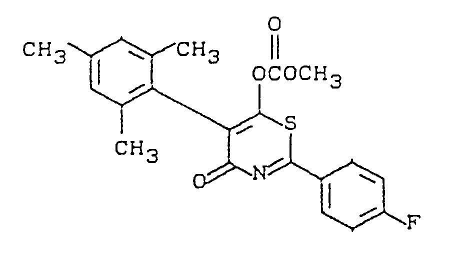

Zu 3,4 g (10 mmol) 2-(4-Fluorphenyl)-6-hydroxy-5-(2,4,6-trimethyl-phenyl)-1,3-thiazin-4-on in 50 ml Ethylacetat tropft man 1,0 g (10 mmol) Triethylamin und darauf 0,94 g (10 mmol) Chlorameisensäuremethylester in 10 ml Ethylacetat bei 0°C. Man rührt 8 Stunden bei 20°C, filtriert, wäscht die organische Phase mit halbkonzentrierter Kochsalzlösung, trocknet über Natriumsulfat und dampft das Lösungsmittel im Vakuum ab. Der Rückstand wird an Kieselgel (35 bis 70 µm) mit Toluol/Aceton (20:1 Volumenteile) chromatographiert. Man erhält 3,1 g 2-(4-Fluorphenyl)-6-methoxycarbonyloxy-5-(2,4,6-trimethylphenyl)-1,3-thiazin-4-on (Ausbeute: 78 % der Theorie). Fp. = 129 bis 131°C.

In entsprechender Weise zu den Herstellungsbeispielen und gemäß den allgemeinen Angaben der Beschreibung zur Herstellung erhält man die in den nachfolgenden Tabellen 1 bis 3 formelmäßig aufgeführten 5-Aryl-1,3-thiazin-Derivate der allgemeinen Formel (I).

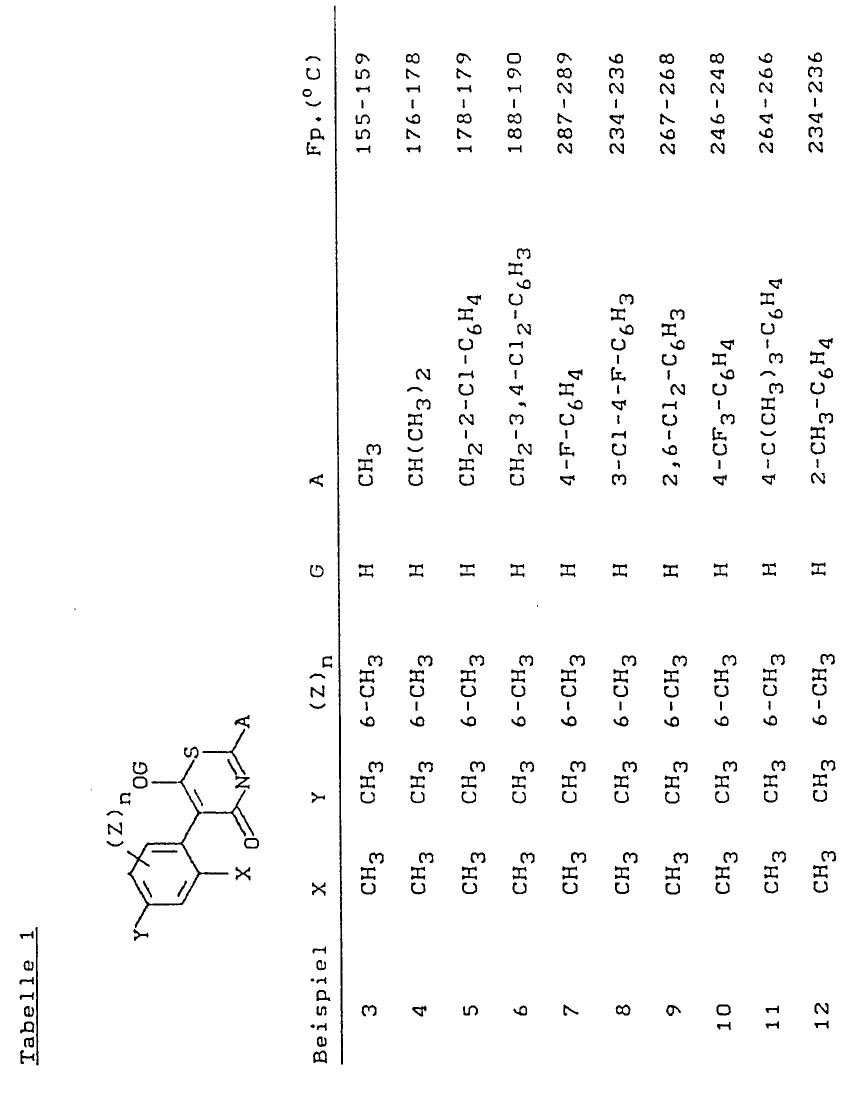

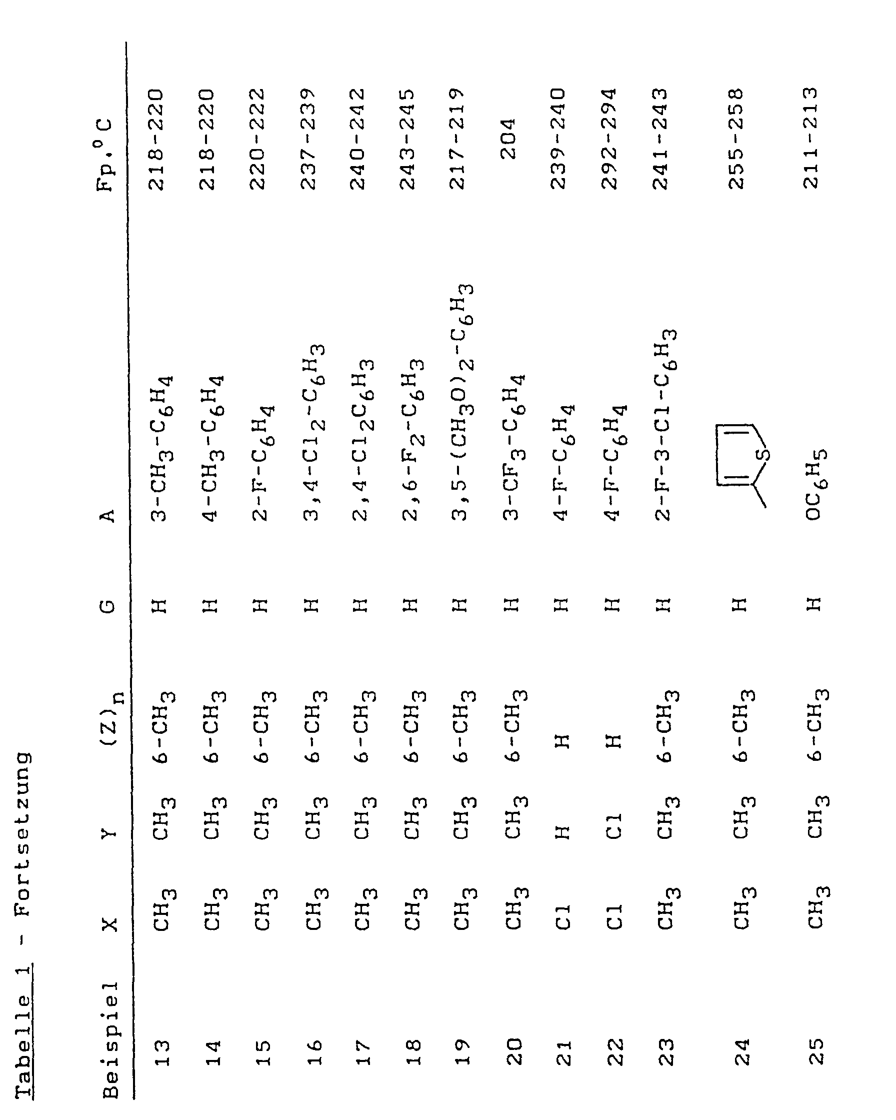

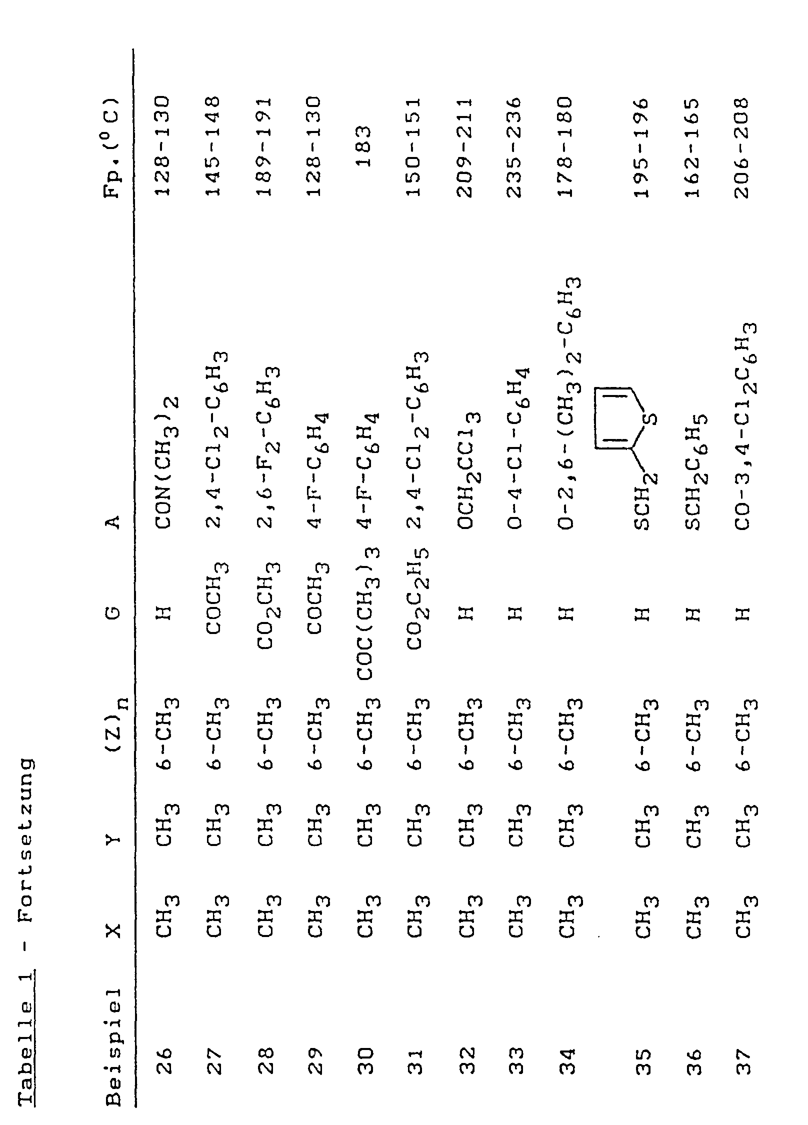

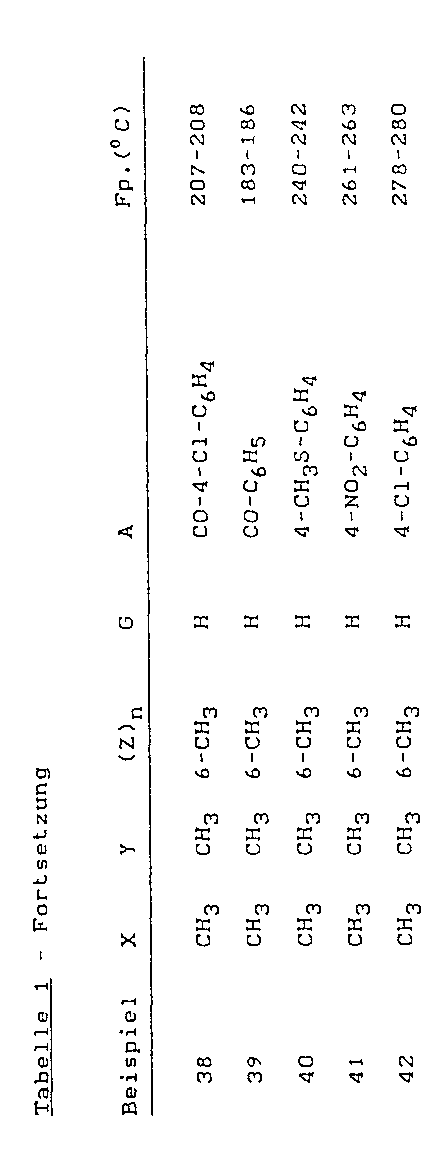

### Herstellung der Ausgangsverbindungen der Formel (III)

### Beispiel (III-1)

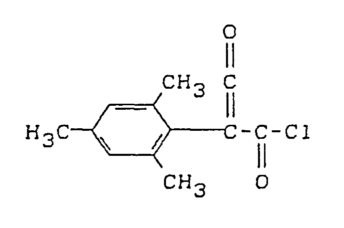

444,5 g (2 Mol) Mesitylmalonsäure werden in 1000 ml Methylcyclohexan bei 75-80°C suspendiert und 714 g (6 Mol) Thionylchlorid innerhalb von 3 Stunden zugetropft. Dann wird langsam weiter erwärmt und 8 Stunden unter Rückflußkühlung bei 110-120°C Badtemperatur nachgerührt.

Überschüssiges Thionylchlorid wird zusammen mit dem Lösungsmittel bei 10 mbar bis 80°C Badtemperatur abdestilliert, der Rückstand nach dem Erkalten mit der 3-fachen Mengen Petrolether verdünnt, filtriert, eingeengt und destilliert.

Man erhält 373 g (84 % der Theorie) Mesitylchlorcarbonylketen vom Siedepunkt 96°/0,45 mbar.

In analoger Weise zu Beispiel (III-1) und unter Berücksichtigung der Angaben in der Beschreibung zu den erfindungsgemäßen Verfahren, können die übrigen Ausgangsverbindungen der Formel (III) hergestellt werden.

### Beispiel A

### Phaedon-Larven-Test

### Lösungsmittel: 7 Gewichtsteile Dimethylformamid

### Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käferlarven abgetötet wurden; 0 % bedeutet, daß keine Käfer-Larven abgetötet wurden.

Bei diesem Test zeigten z.B, die Verbindungen der Herstellungsbeispiele 1,7, 10, 29,2 bei einer beispielhaften Konzentration von 0,1 % nach 7 Tagen eine Abtötung von 100 %.

### Beispiel B

### Plutella-Test

### Lösungsmittel: 7 Gewichtsteile Dimethylformamid

### Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind,

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigten z.B. die Verbindungen der Herstellungsbeispiele 2, 7, 10, 29, 30 bei einer beispielhaften Wirkstoffkonzentration von 0,1 % nach 7 Tagen eine Abtötung von 100 %.

### Beispiel C

### Nephotettix-Test

### Lösungsmittel: 7 Gewichtsteile Dimethylformamid

### Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der grünen Reiszikade (Nephotettix cincticeps) besetzt, solange die Keimlinge noch feucht sind.

Nach der gewünschten Zeit wird die Abtötung in % bestimmt. Dabei bedeutet 100 %, daß alle Zikaden abgetötet wurden; 0 % bedeutet, daß keine Zikaden abgetötet wurden.

Bei diesem Test zeigten z.B. die Verbindungen der Herstellungsbeispiele 2, 7, 12, 15, 29, 30 bei einer beispielhaften Wirkstoffkonzentration von 0,1 % nach 6 Tagen eine Abtötung von 100 %.

### Beispiel D

### Heliothis virescens-Test

### Lösungsmittel: 7 Gewichtsteile Dimethylformamid

### Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Sojatriebe (Glycine max) werden durch Tauchen in die Wirkstoffzubereitung der gewünschten Konzentration behandelt und mit der Tabakknospenraue (Heliothis virescens) besetzt, solange die Blätter noch feucht sind.

Nach der gewünschten Zeit wird die Abtötung in % bestimmt, Dabei bedeutet 100 %, daß alle Raupen abgetötet wurden; 0,% bedeutet, daß keine Raupen abgetötet wurden.

Bei diesem Test zeigten z.B. die Verbindungen der Herstellungsbeispiele eine gute Wirksamkeit.

### Beispiel E

### Pre-emergence-Test

### Lösungsmittel: 5 Gewichtsteile Aceton

### Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen, Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- 0 % =: keine Wirkung (wie unbehandelte Kontrolle)
- 100 % =: totale Vernichtung

Bei diesem Test wurden mit einer beispielhaften Aufwandmenge von 500 bis 1.000 g/ha bei einer guten bis sehr guten Verträglichkeit durch Rüben die folgenden Ergebnisse erhalten:

| Pflanze | % Wirkung | Verbindung des Herstellungsbeispieles Nr. |
|---|---|---|
| Alopecurus | 95 | 1, 7, 29 |
| Cynodon | 100 | 1, 7, 29 |
| Echinochloa | 90 - 100 | 1, 7, 29 |
| Lolium | 95 - 100 | 1, 7, 29 |
| Poa | 80 - 100 | 1, 7, 29 |

## Patentansprüche

1. Substituierte 5-Aryl-1,3-thiazin-Derivate der allgemeinen Formel (I) 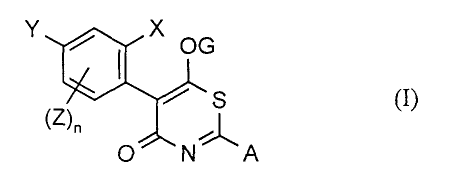 in welcher
A für gegebenenfalls durch Halogen substituierte Reste aus der Reihe C₁-C₈-Alkyl, C₃-C₈-Alkenyl, C₃-C₈-Alkinyl und C₃-C₈-Cycloalkyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyl, Naphthyl, Hetaryl, Phenyl-C₁-C₄-alkyl und Hetaryl-C₁-C₄-alkyl oder für gegebenenfalls durch Halogen substituierte Reste aus der Reihe C₁-C₆-Alkoxy, C₃-C₆-Alkenyloxy, C₃-C₆-Alkinyloxy und C₃-C₆-Cycloalkoxy; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenyloxy, Naphthyloxy, Phenyl-C₁-C₂-alkoxy, Thienyloxy, Furyloxy, Thiazolyloxy, Pyridyloxy und Pyrazolyloxy oder für gegebenenfalls durch Halogen substituierte Reste aus der Reihe C₁-C₆-Alkylthio, C₃-C₆-Alkenylthio, C₃-C₆-Alkinylthio und C₃-C₆-Cycloalkylthio, für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituierte Reste aus der Reihe Phenylthio, Naphthylthio, Phenyl-C₁-C₂-alkylthio, Thienylthio, Thienyl-C₁-C₂-alkylthio, Furylthio, Thiazolylthio, Pyridylthio und Pyrazolylthio oder für die Gruppen
COR¹, CSR¹, CO₂-R¹, 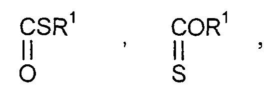 CS₂R¹, CN, CONR¹R² und CSNR¹R² steht, wobei
R¹ und R² unabhängig voneinander für gegebenenfalls durch Halogen substituierte Reste aus der Reihe C₁-C₆-Alkyl und C₃-C₆-Alkenyl; für gegebenenfalls durch Halogen, Nitro, C₁-C₄-Alkyl, C₁-C₄-Alkoxy, C₁-C₄-Alkylthio, C₁-C₄-Halogenalkyl, C₁-C₄-Halogenalkoxy und/oder CN substituiertes Phenyl stehen,
X für Fluor, Chlor, Brom, Methyl, Ethyl, n- und i-Propyl, Methoxy oder Ethoxy steht,
Y für Wasserstoff, Fluor, Chlor, Brom, C₁-C₄-Alkyl oder Trifluormethyl steht,
Z für Wasserstoff, Fluor, Chlor, Brom, C₁-C₄-Alkyl, Methoxy oder Ethoxy steht,
n für 1 steht,
G für Wasserstoff, -COR³ oder COOR⁴ steht,
R³ für gegebenenfalls durch Fluor und/oder Chlor substituierte Reste aus der Reihe C₁-C₁₄-Alkyl, C₂-C₁₄-Alkenyl, C₁-C₄-Alkoxy-C₁-C₆-alkyl, C₁-C₄-Alkylthio-C₁-C₆-alkyl, C₁-C₄-Polyalkoxy-C₁-C₄-alkyl und gegebenenfalls durch 1 oder 2 Sauerstoff- und/oder Schwefelatome unterbrochenes C₃-C₆-Cycloalkyl; für gegebenenfalls durch Fluor, Chlor, Brom, Nitro, C₁-C₃-Alkyl, Methoxy, Ethoxy, Trifluormethyl und/oder Trifluormethoxy substituiertes Phenyl, für gegebenenfalls durch Fluor, Chlor, Brom, C₁-C₃-Alkyl, Methoxy, Ethoxy, Trifluormethyl und/oder Trifluormethoxy substituiertes Phenyl-C₁-C₃-alkyl; für gegebenenfalls durch Fluor, Chlor, Brom, Methyl und/oder Ethyl substituierte Reste aus der Reihe Pyridyl, Pyrimidyl, Thiazolyl und Pyrazolyl;
R⁴ für gegebenenfalls durch Fluor und/oder Chlor substituierte Reste aus der Reihe C₁-C₁₄-Alkyl, C₂-C₁₄-Alkenyl, C₁-C₄-Alkoxy-C₁-C₆-alkyl, C₁-C₄-Polyalkoxy-C₁-C₆-alkyl oder für gegebenenfalls durch Fluor, Chlor, Nitro, C₁-C₃-Alkyl, Methoxy, Ethoxy und/oder Trifluormethyl substituierte Reste aus der Reihe Phenyl und Benzyl steht,
wobei als Hetarylreste Pyrryl, Furyl, Thienyl, Thiazolyl, Pyridyl, Pyrazolyl und Pyrimidinyl in Frage kommen.

2. Verbindungen der Formel (I) gemäß Anspruch 1, in welcher
A für gegebenenfalls durch Halogen substituierte Reste aus der Reihe Phenyl, Phenyloxy, Phenylthio steht,
X für Methyl steht,
Y für Methyl steht,
Z für Methyl steht,
n für 1 steht,
G für Wasserstoff steht.

3. Verfahren zur Herstellung von substituierten 5-Aryl-1,3-thiazin-Derivaten der allgemeinen Formel (I) gemäß Anspruch 1, **dadurch gekennzeichnet, daß** man
a) zur Herstellung der Verbindungen, in welchen G für Wasserstoff steht,
Thioamide der allgemeinen Formel (II) 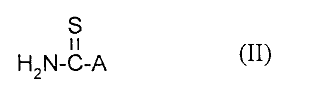 in welcher
A die oben angegebene Bedeutung hat,
mit Ketensäurehalogeniden der allgemeinen Formel (III) 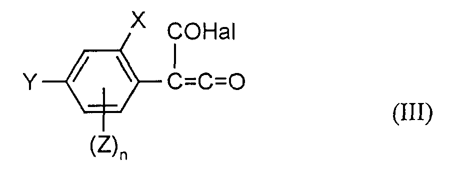 in welcher
X, Y, Z und n die oben angegebenen Bedeutungen haben und
Hal für Halogen steht,
in Gegenwart eines Verdünnungsmittels und gegebenenfalls in Gegenwart eines Säureakzeptors umsetzt; und
b) zur Herstellung der Verbindungen der allgemeinen Formel (I), in welcher G für -COR³ steht,
Verbindungen der allgemeinen Formel (I), in welchen G für Wasserstoff steht (erhältlich nach Variante a))
α) mit Säurehalogeniden der allgemeinen Formel (IV) 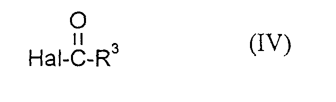 in welcher
R³ die oben angegebene Bedeutung hat und
Hal für Halogen steht, oder
β) mit Carbonsäureanhydriden der allgemeinen Formel (V) 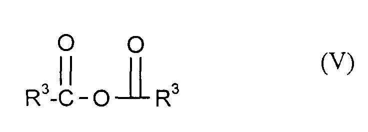 in welcher
R³ die oben angegebene Bedeutung hat,
gegebenenfalls in Gegenwart eines Verdünnungsmittels und gegebenenfalls in Gegenwart eines Säurebindemittels umsetzt; und
c) zur Herstellung der Verbindungen der allgemeinen Formel (I), in welchen G für COOR⁴ und R⁴ die oben angegebene Bedeutung besitzt,
Verbindungen der allgemeinen Formel (I), in welchen G für Wasserstoff steht (erhältlich nach Variante a)),
mit Verbindungen der allgemeinen Formel (VI) 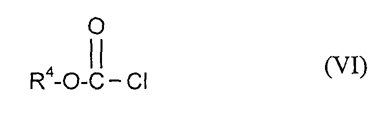
in welcher
R⁴ die oben angegebenen Bedeutungen hat,
gegebenenfalls in Gegenwart eines Verdünnungsmittels und gegebenenfalls in Gegenwart eines Säurebindemittels umsetzt.

4. Schädlingsbekämpfungsmittel, **gekennzeichnet durch** einen Gehalt an mindestens einer Verbindung der Formel (I) gemäß Anspruch 1.

5. Verwendung von Verbindungen der Formel (I) gemäß Anspruch 1 zur Bekämpfung von Schädlingen im Pflanzenschutz, Haushaltsbereich, Hygienebereich und Vorratsschutz.

6. Verfahren zur Bekämpfung von Schädlingen im Pflanzenschutz, Haushaltsbereich, Hygienebereich und Vorratsschutz, **dadurch gekennzeichnet, daß** man Verbindungen der Formel (I) gemäß Anspruch 1 auf die Schädlinge und/oder ihren Lebensraum einwirken läßt.

7. Verfahren zur Herstellung von Schädlingsbekämpfungsmitteln, **dadurch gekennzeichnet, daß** man Verbindungen der Formel (I) gemäß Anspruch 1 mit Streckmitteln und/oder oberflächenaktiven Mitteln vermischt.

8. Verwendung von Verbindungen der Formel (I) gemäß Anspruch 1 zur Herstellung von Schädlingsbekämpfungsmitteln zur Bekämpfung von Ekto- und Endoparasiten.

## Claims

1. Substituted 5-aryl-1,3-thiazine derivatives of the general formula (I) 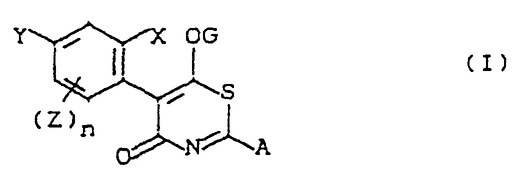 in which
A represents radicals from the series consisting of C₁-C₈-alkyl, C₃-C₈-alkenyl, C₃-C₈-alkinyl, and C₃-C₈-cyclo-alkyl which are optionally substituted by halogen; or represents radicals from the series consisting of phenyl, naphthyl, hetaryl, phenyl-C₁-C₄-alkyl and hetaryl-C₁-C₄-alkyl which are optionally substituted by halogen, nitro, C₁-C₄-alkyl, C₁-C₄-alkoxy, C₁-C₄-alkylthio, C₁-C₄-halogenoalkyl, C₁-C₄-halogenoalkoxy and/or CN, or represents radicals from the series consisting of C₁-C₆-alkoxy, C₃-C₆-alkenyloxy, C₃-C₆-alkinyloxy, and C₃-C₆-cycloalkoxy which are optionally substituted by halogen; or represents radicals from the series consisting of phenyloxy, naphthyloxy, phenyl-C₁-C₂-alkoxy and thienyloxy, furyloxy, thiazolyloxy, pyridyloxy and pyrazolyloxy which are optionally substituted by halogen, nitro, C₁-C₄-alkyl, C₁-C₄-alkoxy, C₁-C₄-alkylthio, C₁-C₄-halogenoalkyl, C₁-C₄-halogenoalkoxy and/or CN; or represents radicals from the series consisting of C₁-C₆-alkylthio, C₃-C₆-alkenylthio, C₃-C₆-alkinylthio and C₃-C₆-cycloalkylthio which are optionally substituted by halogen, or represents radicals from the series consisting of phenylthio, naphthylthio, phenyl-C₁-C₂-alkylthio, thienylthio, thienyl-C₁-C₂-alkylthio, furylthio, thiazolylthio, pyridylthio and pyrazolylthio which are optionally substituted by halogen, nitro, C₁-C₄-alkyl, C₁-C₄-alkoxy, C₁-C₄-alkylthio, C₁-C₄-halogenoalkyl, C₁-C₄-halogenoalkoxy and/or CN, or represents the groups COR¹, CSR¹, CO₂-R¹ 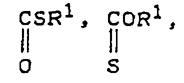 CS₂R¹, CN, CONR¹R² and CSNR¹R², where
R¹ and R² independently of one another represent radicals from the series consisting of C₁-C₆-alkyl and C₃-C₆-alkenyl. which are optionally substituted by halogen; or represent phenyl which is optionally substituted by halogen, nitro, C₁-C₄-alkyl C₁-C₄-alkoxy, C₁-C₄-alkylthio C₁-C₄-halogenoalkyl, C₁-C₄-halogenoalkoxy and/or CN
X represents fluorine, chlorine, bromine, methyl, ethyl, n- and i-propyl, methoxy or ethoxy,
Y represents hydrogen, fluorine, chlorine, bromine, C₁-C₄-alkyl or trifluoromethyl,
Z represents hydrogen, fluorine, chlorine, bromine, C₁-C₄-alkyl, methoxy or ethoxy,
n represents 1,
G represents hydrogen, -COR³ or COOR⁴,
R³ represents optionally fluorine- and/or chlorine-substituted radicals from the series consisting of C₁-C₁₄-alkyl, C₂-C₁₄-alkenyl, C₁-C₄-alkoxy-C₁-C₆-alkyl, C₁-C₄-alkylthio-C₁-C₆-alkyl, C₁-C₄-polyalkoxy-C₁-C₆-alkyl and C₃-C₆-cycloalkyl, optionally interrupted by 1 or 2 oxygen and/or sulphur atoms; or represents phenyl which is optionally substituted by fluorine, chlorine, bromine, nitro, C₁-C₃-alkyl, methoxy, ethoxy, trifluoromethyl and/or trifluoromethoxy; or represents phenyl-C₁-C₃-alkyl which is optionally substituted by fluorine, chlorine, bromine, C₁-C₃-alkyl, methoxy, ethoxy, trifluoromethyl and/or trifluoromethoxy; or represents radicals from the series consisting of pyridyl, pyrimidyl, thiazolyl and pyrazolyl which are optionally substituted by fluorine, chlorine, bromine, methyl and/or ethyl;
R⁴ represents radicals from the series consisting of C₁-C₁₄-alkyl, C₂-C₁₄-alkenyl, C₁-C₄-alkoxy-C₁-C₆-alkyl and C₁-C₄-polyalkoxy-C₁-C₆-alkyl which are optionally substituted by fluorine and/or chlorine, or represents radicals from the series consisting of phenyl and benzyl which are optionally substituted by fluorine, chlorine, nitro, C₁-C₃-alkyl, methoxy, ethoxy and/or trifluoromethyl,
suitable hetaryl radicals being pyrryl, furyl, thienyl, thiazolyl, pyridyl, pyrazolyl and pyrimidinyl.

2. Compounds of the formula (I) according to Claim 1, in which
A represents radicals from the series consisting of phenyl, phenyloxy and phenylthio which are optionally substituted by halogen,
X represents methyl,
Y represents methyl,
Z represents methyl,
n represents 1,
G represents hydrogen.

3. Process for the preparation of substituted 5-aryl-1,3-thiazine derivatives of the general formula (I) according to Claim 1, **characterized in that**
a) to prepare the compounds in which G represents hydrogen,
thioamides of the general formula (II) 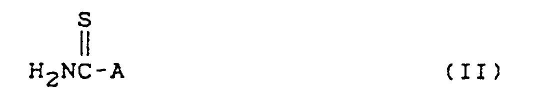 in which
A has the abovementioned meaning
are reacted with ketenic acid halides of the general formula (III) 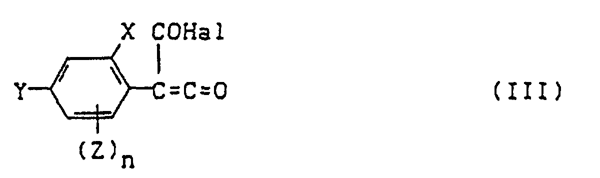 in which
X, Y, Z and n have the abovementioned meanings and
Hal represents halogen,
in the presence of a diluent and if appropriate in the presence of an acid acceptor; and
b) to prepare the compounds of the general formula (I) in which G represents -COR³,
compounds of the general formula (I) in which G represents hydrogen (which can be obtained by variant a)) are reacted
α) with acid halides of the general formula (IV) 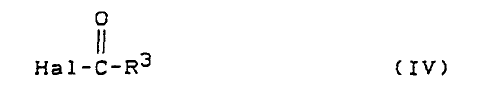 in which
R³ has the abovementioned meaning and
Hal represents halogen, or
β) with carboxylic anhydrides of the general formula (V) 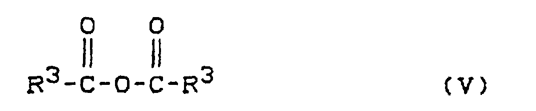 in which
R³ has the abovementioned meaning,
if appropriate in the presence of a diluent and if appropriate in the presence of an acid-binding agent; and
c) to prepare the compounds of the general formula (I) in which G represents COOR⁴ and R⁴ has the abovementioned meaning,
compounds of the general formula (I) in which G represents hydrogen (which can be obtained by variant a))
are reacted with compounds of the general formula (VI) 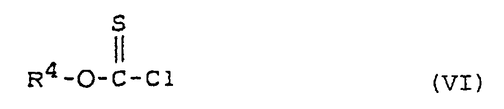
in which
R⁴ has the abovementioned meanings,
if appropriate in the presence of a diluent and if appropriate in the presence of an acid-binding agent.

4. Pesticides, **characterized in that** they contain at least one compound of the formula (I) according to Claim 1.

5. Use of compounds of the formula (I) according to Claim 1 for combating pests in plant protection, on domestic premises, in the hygiene field and the protection of stored products.

6. Method of combating pests in plant protection, on domestic premises, in the hygiene field and the protection of stored products, **characterized in that** compounds of the formula (I) according to Claim 1 are allowed to act on the pests and/or their environment.

7. Process for the preparation of pesticides, **characterized in that** compounds of the formula (I) according to Claim 1 are mixed with extenders and/or surface-active agents.

8. Use of compounds of the formula (I) according to Claim 1 for the preparation of pesticides for combating ecto- and endoparasites.

## Revendications

1. Dérivés substitués de 5-aryl-1,3-thiazines de formule générale (I) 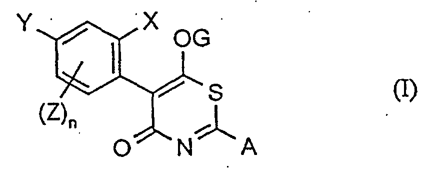 dans laquelle
A représente des restes, éventuellement substitués par un halogène, de la série alkyle en C₁ à C₈, alcényle en C₃ à C₈, alcynyle en C₃ à C₈ et cycloalkyle en C₃ à C₈ ; des restes, éventuellement substitués par un radical halogéno, nitro, alkyle en C₁ à C₄, alkoxy en C₁ à C₄, alkylthio en C₁ à C₄, halogénalkyle en C₁ à C₄, halogénalkoxy en C₁ à C₄ et/ou CN, de la série phényle, naphtyle, hétaryle, phényl-(alkyle en C₁ à C₄) et hétaryl-(alkyle en C₁ à C₄) ou des restes, éventuellement substitués par un halogène, de la série alkoxy en C₁ à C₆, alcényloxy en C₃ à C₆, alcynyloxy en C₃ à C₆ et cycloalkoxy en C₃ à C₆ ; des restes, éventuellement substitués par un radical halogéno, nitro, alkyle en C₁ à C₄, alkoxy en C₁ à C₄, alkylthio en C₁ à C₄, halogénalkyle en C₁ à C₄, halogénalkoxy en C₁ à C₄ et/ou CN, de la série phényloxy, naphtyloxy, phényl-(alkoxy en C₁ ou C₂), thiényloxy, furyloxy, thiazolyloxy, pyridyloxy et pyrazolyloxy ou des restes, éventuellement substitués par un halogène, de la série alkylthio en C₁ à C₆, alcénylthio en C₃ à C₆, alcynylthio en C₃ à C₆ et cycloalkylthio en C₃ à C₆, des restes, éventuellement substitués par un radical halogéno, nitro, alkyle en C₁ à C₄, alkoxy en C₁ à C₄, alkylthio en C₁ à C₄, halogénalkyle en C₁ à C₄, halogénalkoxy en C₁ à C₄ et/ou CN, de la série phénylthio, naphtylthio, phényl-(alkylthio en C₁ ou C₂, thiénylthio, thiényl-(alkylthio en C₁ ou C₂), furylthio, thiazolylthio, pyridylthio et pyrazolylthio ou les groupes
COR¹, CSR¹, CO₂-R¹, 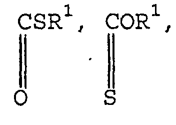 CS₂R¹, CN, CONR¹R² et CSNR¹R²,
dans lesquels
R¹ et R² représentent indépendamment l'un de l'autre des restes, éventuellement substitués par un halogène, de la série alkyle en C₁ à C₆ et alcényle en C₃ à C₆ ; un groupe phényle éventuellement substitué par un radical halogéno, nitro, alkyle en C₁ à C₄, alkoxy en C₁ à C₄, alkylthio en C₁ à C₄, halogénalkyle en C₁ à C₄, halogénalkoxy en C₁ à C₄ et/ou CN,
X représente le fluor, le chlore, le brome, un groupe méthyle, éthyle, n-propyle, iso-propyle, méthoxy ou éthoxy,
Y représente l'hydrogène, le fluor, le chlore, le brome, un groupe alkyle en C₁ à C₄ ou trifluorométhyle,
Z représente l'hydrogène, le fluor, le chlore, le brome, un groupe alkyle en C₁ à C₄, méthoxy ou éthoxy,
n est égal à 1,
G représente l'hydrogène, un groupe -COR³ ou COOR⁴,
R³ représente des restes, éventuellement substitués par du fluor et/ou du chlore, de la série alkyle en C₁ à C₁₄, alcényle en C₂ à C₁₄, (alkoxy en C₁ à C₄)-(alkyle en C₁ à C₆), (alkylthio en C₁ à C₄)-(alkyle en C₁ à C₆), poly-(alkoxy en C₁ à C₄)-(alkyle en C₁ à C₄) et un groupe cycloalkyle en C₃ à C₆ éventuellement interrompu par un ou deux atomes d'oxygène et/ou de soufre ; un groupe phényle éventuellement substitué par un radical fluoro, chloro, bromo, nitro, alkyle en C₁ à C₃, méthoxy, éthoxy, trifluorométhyle et ou trifluorométhoxy, un groupe phényl-(alkyle en C₁ à C₃) éventuellement substitué par un radical fluoro, chloro, bromo, alkyle en C₁ à C₃, méthoxy, éthoxy, trifluorométhyle et/ou trifluorométhoxy ; des restes, éventuellement substitués par un radical fluoro, chloro, bromo, méthyle et/ou éthyle, de la série pyridyle, pyrimidyle, thiazolyle et pyrazolyle ;
R⁴ représente des restes, éventuellement substitués par du fluor et/ou du chlore, de la série alkyle en C₁ à C₁₄, alcényle en C₂ à C₁₄, (alkoxy en C₁ à C₄)-(alkyle en C₁ à C₆), poly-(alkoxy en C₁ à C₄)-(alkyle en C₁ à C₆) ou des restes, éventuellement substitués par un radical fluoro, chloro, nitro, alkyle en C₁ à C₃, méthoxy, éthoxy et/ou trifluorométhyle, de la série phényle et benzyle,
en considérant comme restes hétaryle les restes pyrryle, furyle, thiényle, thiazolyle, pyridyle, pyrazolyle et pyrimidinyle.

2. Composés de formule (I) suivant la revendication 1, dans laquelle
A représente des restes, éventuellement substitués par un halogène, de la série phényle, phényloxy, phénylthio,
X est un groupe méthyle,
Y est un groupe méthyle,
Z est un groupe méthyle,
n est éqal à 1,
G représente l'hydrogène.

3. Procédé de production de dérivés substitués de 5-aryl-1,3-thiazines de formule générale (I) suivant la revendication 1, **caractérisé en ce que** :
a) pour la production des composés dans lesquels G représente l'hydrogène,
on fait réagir des thioamides de formule générale (II) 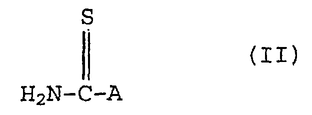 dans laquelle
A a la définition indiquée ci-dessus,
avec des halogénures d'acides cétène-carboxyliques de formule générale (III) 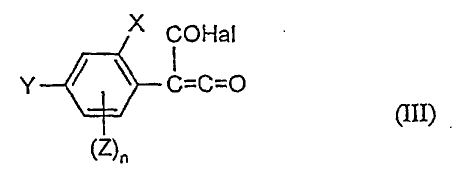 dans laquelle
X, Y, Z et n ont les définitions indiquées ci-dessus et
Hal représente un halogène,
en présence d'un diluant et en présence éventuelle d'un accepteur d'acide ; et
b) pour la production des composés de formule générale (I), dans laquelle G représente -COR³,
on fait réagir des composés de formule générale (I), dans lesquels G représente l'hydrogène (obtenus selon la variante (a))
α) avec des halogénures d'acides de formule générale (IV) 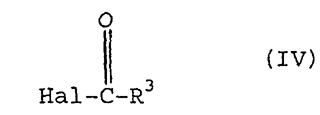 dans laquelle
R³ a la définition indiquée ci-dessus et
Hal représente un halogène, ou bien
β) avec des anhydrides d'acides carboxyliques de formule générale (V) 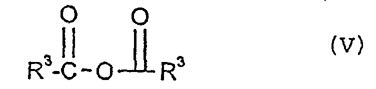 dans laquelle
R³ a la définition indiquée ci-dessus,
le cas échéant en présence d'un diluant et en présence éventuelle d'un accepteur d'acide ; et
c) pour la production des composés de formule générale (I), dans lesquels G représente COOR⁴ et R⁴ a la définition indiquée ci-dessus,
on fait réagir des composés de formule générale (I), dans lesquels G représente l'hydrogène (obtenus selon la variante (a))
avec des composés de formule générale (VI) 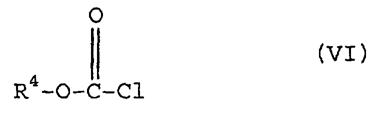 dans laquelle
R⁴ a la définition indiquée ci-dessus,
le cas échéant en présence d'un diluant et en présence éventuelle d'un accepteur d'acide.

4. Compositions pesticides, **caractérisées par** une teneur en au moins un composé de formule (I) suivant la revendication 1.

5. Utilisation de composés de formule (I) suivant la revendication 1 pour combattre des parasites dans la protection des plantes, dans le domaine domestique, dans le domaine de l'hygiène et dans la protection des denrées entreposées.

6. Procédé pour combattre des parasites dans la protection des plantes, le domaine domestique, le domaine de l'hygiène et la protection des denrées entreposées, **caractérisé en ce qu'**on fait agir des composés de formule (I) suivant la revendication 1 sur les parasites et/ou sur leur milieu.

7. Procédé de préparation de compositions pesticides, **caractérisé en ce qu'**on mélange des composés de formule (I) suivant la revendication 1 avec des diluants et/ou des agents tensio-actifs.

8. Utilisation de composés de formule (I) suivant la revendication 1 pour la préparation de compositions pesticides destinées à combattre des ectoparasites et des endoparasites.
